**Europäisches Patentamt**

**European Patent Office**

**Office européen des brevets**

(19)

(11) Publication number: **0 101 148**
**B1**

(12) # EUROPEAN PATENT SPECIFICATION

(45) Date of publication of patent specification: **23.12.87**

(21) Application number: **83302409.4**

(22) Date of filing: **28.04.83**

(51) Int. Cl.⁴: **C 07 D 277/42,**
**C 07 D 501/06, C 07 D 501/56**

(54) Thiazole derivative, process for its preparation and use in the preparation of beta-lactam antibiotics.

(30) Priority: **29.04.82 GB 8212491**

(43) Date of publication of application:
**22.02.84 Bulletin 84/08**

(45) Publication of the grant of the patent:
**23.12.87 Bulletin 87/52**

(84) Designated Contracting States:
**AT BE CH DE FR GB IT LI NL SE**

(56) References cited:
**EP-A-0 053 542**
**US-A-4 258 041**

**Chemical Abstracts vol. 96, no. 19, 10 May
1982, Columbus, Ohio, USA; page 729, colm. 1,
abstract no. 162430z**

(73) Proprietor: **GLAXO GROUP LIMITED
Clarges House 6-12 Clarges Street
London W1Y 8DH (GB)**

(72) Inventor: **Trivedi, Harish Shivprasad
93 Ennismore Avenue
Greenford Middlesex (GB)**

(74) Representative: **Holmes, Michael John et al
Frank B. Dehn & Co. European Patent Attorneys
Imperial House 15-19 Kingsway
London, WC2B 6UZ, (GB)**

# 0 101 148

**Description**

This invention relates to improvements in or relating to the manufacture of antibiotics. More particularly it relates to a new compound that is of value in the synthesis of certain β-lactam antibiotics, for example, cephalosporine and 2-oxoazetidines.

A number of antibiotic compounds are now known which have a *syn* 2 - (2 - aminothiazol - 4 - yl) - 2 - (substituted oxyimino)acetamido side-chain in the 7-position. One such antibiotic, (6R,7R) - 7 - [(Z) - 2 - (2 - aminothiazol - 4 - yl) - 2 - (2 - carboxyprop - 2 - oxyimino)acetamido] - 3 - (1 - pyridiniummethyl)-ceph - 3 - em - 4 - carboxylate, hereinafter called "ceftazidime" is described, *inter alia*, in our U.S. Patent Specification No. 4,258,041 and details of its preparation are given therein.

One general method for preparing ceftazidime involves building up the 7-position side chain through a series of reactions to form the side-chain acid or a reactive derivative thereof followed by coupling with the 76-amino cephalosporin nucleus. A series of such reactions for the preparation of a protected form of the 7-side chain acid is described in Preparations 1 to 4 of the above-mentioned U.S. Patent Specification. The protected side-chain acid described in these preparations is (Z-2-(2-t-butoxycarbonylprop-2-oxyimino)-2-(2-triphenylmethylaminothiazol-4-yl)acetic acid.

In one method specifically described in U.S. Patent No. 4,258,041 for the production of ceftazidime the side chain acid is converted into an acid chloride hydrochloride by reaction with $PCl_5$ and this is reacted *in situ* with the appropriate 7β-aminocephalosporin. Prior to the coupling reaction it is necessary to remove from the solution containing the acid chloride hydrochloride any excess phosphorus halide compounds, e.g. phosphorus oxychloride, which may interfere with the acylation step, by treating the reaction mixture with water. However, the water also hydrolyses some of the acid chloride hydrochloride with the result that a variable and unknown amount of the reactive compound is available for the subsequent coupling reaction. This leads to reduced and inconsistent yields of the coupled product, which additionally tends to contain impurities.

We have now found that (Z)-2-(2-t-butoxycarbonylprop-2-oxyimino)-2-(2-triphenylmethylamino-thiazol-4-yl)acetyl chloride hydrochloride may be readily and consistently isolated with a high degree of purity. This compound has also been found to exhibit very good stability, both on storage, even at a high temperature over extended periods, and on mechanical handling. We have also found that the compound may be isolated as a free-flowing particulate material.

Viewed from one aspect, therefore, we provide isolated (Z)-2-(2-t-butoxycarbonylprop-2-oxyimino)-2-(2-triphenylmethylaminothiazol-4-yl)acetyl chloride hydrochloride.

The isolated acid chloride hydrochloride may advantageously be obtained in substantially pure form. The isolated acid chloride hydrochloride according to the invention may preferably be obtained in crystalline form. The new crystalline acid chloride hydrochloride has been characterised by its X-ray diffraction properties. The product of the following Example 3 was used to obtain a Debye-Scherrer powder diffraction photograph by exposure for 12 hours to $CoK_q$ radiation and a second photograph by exposure for 3 hours to $CuK_q$ radiation. The results obtained are shown in the following Table; all "d" values being given in Ångström units, Å.

### TABLE

| 'd' value | Intensity | 'd' value | Intensity |
|-----------|-----------|-----------|-----------|
| 15.55 | s | 4.26 | s |
| 13.71 | vw | 4.10 | w |
| 8.98 | vs | 3.85 | s |
| 6.52 | m | 3.68 | md |
| 6.24 | m | 3.30 | md |
| 5.92 | wd | 3.15 | wd |
| 5.47 | md | 2.87 | wd |
| 4.61 | m | 2.60 | vwd |
| 4.43 | m | 2.54 | vwd |

s=strong; m=medium; w=weak; v=very; d=diffuse.

The isolated acid chloride hydrochloride may be used in a coupling reaction with an amino β-lactam to give higher and more consistent yields of good quality coupled product than does the reaction proceeding

2

via the *in situ* activated compounds and hence a higher yield of the subsequently deprotected final product is obtained. Separating the activation step from the coupling step and isolating the activated intermediate enables the quantity of acid chloride hydrochloride used in the acylation to be accurately measured and hence allows better control over the reaction. In addition, by using the isolated acid chloride hydrochloride, impurities in the coupled product arising from activating reagents are virtually eliminated. The isolated acid chloride hydrochloride thus possesses considerable advantages in the production of β-lactam antibiotics containing the (Z)-2-(2-aminothiazol-4-yl)-2-(2-carboxyprop-2-oxyimino)acetamido side chain.

In a further aspect, the invention provides a process for the preparation of the isolated acid chloride hydrochloride which comprises reacting (Z)-2-(2-t-butoxycarbonylprop-2-oxyimino)-2-(2-triphenylmethyl-aminothiazol-4-yl)acetic acid or a solvate or salt thereof with a chlorinating agent serving to convert the carboxyl group to a chlorocarbonyl group, hydrogen chloride being liberated in the reaction or otherwise introduced whereby the desired acid chloride hydrochloride is formed and is subsequently isolated.

The chlorinating agents normally used to convert carboxylic acids to acid chlorides liberate hydrogen chloride in the reaction. Such reagents include, for example, phosphorus pentachloride, phosphorus trichloride and thionyl chloride. The reaction is conveniently effected in the presence of an anhydrous organic solvent which is inert under the reaction conditions employed. Examples of solvents which may be used include anhydrous halogenated hydrocarbons such as dichloromethane. The reaction may be conveniently effected at a temperature of −50 to +50°C, preferably below 0°C e.g. between −20 and 0°C.

Following reaction with the chlorinating agent, the desired product may be isolated directly from the reaction medium e.g. by precipitation. In order to maximise the yield of desired product, it may be advantageous substantially to complete precipitation by contacting the reaction mixture with a further anhydrous organic solvent, such as di-isopropyl ether, in which the acid chloride hydrochloride is less soluble.

In general, the preparation of the compound of the invention should be effected under anhydrous conditions in order to avoid hydrolysis of the product. It is also desirable to avoid the use of solvents which contain groups such as hydroxyl, amino or mercapto groups, which may react with the acid chloride hydrochloride.

The (Z)-2-(2-t-butoxycarbonylprop-2-oxyimino)-2-(triphenylmethylaminothiazol-4-yl)acetic acid from which the acid chloride hydrochloride may be prepared may itself be prepared by a variety of synthetic routes. One such route is described in U.S. Patent Specification No. 4,258,041.

As indicated above, the isolated acid chloride hydrochloride according to the invention may be advantageously used in a coupling reaction with am amino β-lactam compound.

Thus, in a further aspect, the invention provides a process for the preparation of a β-lactam antibiotic compound having a (Z)-2-(2-aminothiazol-4-yl)-2-(2-carboxyprop-2-oxyimino)acetamido side chain which comprises reacting the isolated acid chloride hydrochloride according to the invention with an amino β-lactam compound, followed by removal of protecting groups, whereafter the desired β-lactam antibiotic is recovered. The N-acylation of the amino β-lactam and the subsequent removal of the protecting groups may be effected by conventional methods. For example as described in U.S. Patent No. 4,258,041.

A particularly preferred product of this process is ceftazidime, in which case the starting amino β-lactam compound for the coupling reaction will be 7β-amino-3-(1-pyridiniummethyl)ceph-3-em-4-carboxylate or a salt thereof, preferably the dihydrochloride. The coupling reaction may be effected as described in U.S. Patent No. 4,258,041.

Examples of further β-lactam antibiotic compounds which may be prepared by the coupling reaction include [3S - [3α(Z)4β]] - 3 - [2 - (2 - aminothiazol - 4 - yl) - 2 - (2 - carboxyprop - 2 - oxyimino)acetamido] - 4 - methyl - 2 - oxo - 1 - azetidinesulphonic acid (known by the approved name "azthreonam") which is described in U.K. Patent Specification No. 2071650A, as well as compounds described in our U.K. Patent Specifications Nos. 2029824A, 2024808A, 2027691A, 2040921A, 2036724A, 2037281A, 2043641A and 2046261A, in which the 7-side chain is a (Z) - 2 - (2 - aminothiazol - 4 - yl) - 2 - (2 - carboxyprop - 2 - oxyimino)acetamido moiety.

The following non-limiting Examples serve to illustrate the invention. All temperatures are in °C.

**Example 1**

(Z)-2-(2-t-Butoxycarbonylprop-2-oxyimino)-2-(2-triphenylmethylaminothiazol-4-yl)acetyl chloride hydrochloride

A mixture of phosphorus pentachloride (0.76 g) and dichloromethane (7 ml) was stirred and cooled to −20°, whereupon (Z)-2-(2-t-butoxycarbonylprop-2-oxyimino)-2-(2-triphenylmethylaminothiazol-4-yl)acetic acid (2.07 g) was added. The temperature was maintained at −5° for 30 minutes, and the solution was then added to di-isopropyl ether (25 ml). The precipitate was collected by filtration and washed with di-isopropyl ether and dried to give the *title compound* (1.81 g). $v_{max}$ (Nujol) includes 1785 cm$^{-1}$ (—COCl) $\tau$(CDCl$_3$) −1.9 (—N*H*); 2.68 (trityl protons); 3.58 (aminothiazole ring proton); 8.29 (gem dimethyl protons); 8.52 (t-butyl protons).

**Example 2**

(Z)-2-(2-t-Butoxycarbonylprop-2-oxyimino)-2-(2-triphenylmethylaminothiazol-4-yl)acetyl chloride hydrochloride

(Z)-2-(2-t-Butoxycarbonylprop-2-oxyimino)-2-triphenylmethylaminothiazol-4-yl)acetic acid (80 g) in

3

dichloromethane (300 ml) was stirred and cooled to −15°. Phosphorus pentachloride (30.1 g) was added and the mixture was stirred at −5° for 30 minutes. It was cooled to −10° and di-isopropyl ether (1200 ml) was added over 17 minutes. The precipitate was collected by filtration and washed with di-isopropyl ether to give the *title compound* (81.4 g).

Found C, 60.86; H, 5.22; Cl, 11.3; N, 6.39; P, 0.057; S, 5.03%

$C_{32}H_{33}Cl_2N_3O_4S$ requires C, 61.33; H, 5.3; Cl, 11.32; N, 6.7; P, 0.0; S, 5.11%

Water content: 0.1% (Carl Fischer).

$\tau(CDCl_3)$ values resemble those of Example 1.

Example 3

(Z)-2-(2-t-Butoxycarbonylprop-2-oxyimino)-2-(2-triphenylmethylaminothiazol-4-yl)acetyl chloride hydrochloride

(Z)-2-(2-t-Butoxycarbonylprop-2-oxyimino)-2-(2-triphenylmethylaminothiazol-4-yl)acetic acid (16.5 g) was added to a stirred suspension of phosphorus pentachloride (6.08 g) in dichloromethane (60 ml) at −20°. The temperature rose and the solution was stirred at −5° for 30 minutes. The solution was cooled to −10° and di-isopropyl ether (240 ml) was added over 15 minutes. The precipitated solid was collected by filtration, washed with di-isopropyl ether and dried to give the *title compound* (15.45 g).

Found C, 61; H, 5.32; N, 6.7; P<0.05; Cl, 11.3; S, 5.15%

$C_{32}H_{33}N_3Cl_2O_4S$

Requires C, 61.33; H, 5.3; N, 6.7; Cl, 11.32; S, 5.11%

$\tau(CDCl_3)$ values resemble those of Example 1.

Debye-Scherrer powder diffraction X-ray photographs showed the material to be crystalline.

Example 4

(6R,7R)-7-[(Z)-2-(2-Triphenylmethylaminothiazol-4-yl)-2-(2-t-butoxycarbonylprop-2-oxyimino)acetamido]-3-(1-pyridiniummethyl)ceph-3-em-4-carboxylate, N,N-dimethylforamide solvate

A solution of (Z)-2-(2-t-butoxycarbonylprop-2-oxyimino)-2-(2-triphenylmethylaminothiazol-4-yl)acetyl chloride hydrochloride (8.26 g), prepared as in Example 2 above, in dichloromethane (54 ml) was added over 3 minutes to a stirred mixture of (6R,7R)-7-amino-3-(1-pyridiniummethyl)ceph-3-em-4-carboxylate, dihydrochloride dihydrate (4.8 g) and triethylamine (8.38 ml) in industrial methylated spirits (30 ml) and dichloromethane (30 ml) initially at −20°C. After 30 minutes at 0° this solution was treated with water (135 ml) over 3 minutes. The phases were separated and the organic phase was stirred with water (50 ml) for 5 minutes. The phases were again separated. The combined aqueous washes were extracted with a small volume of dichloromethane and the extract was added to the organic phase. The organic phase was dried ($Na_2SO_4$) and then evaporated under reduced pressure. The residual foam was dissolved in N,N-dimethylformamide (44 ml) and a seed crystal of *title compound* was added. When crystallization was well-advanced di-isopropyl ether (70 ml) was added over 30 minutes to the stirred mixture. The solid was isolated by filtration, washed and dried to give 10.93 g of title compound.

N,N-Dimethylformamide content=17% m/m by gas-liquid chromatographic assay. $[\alpha]_D^{20} -29.8°$ (c=1.0 in methanol). $\tau(DMSO\text{-}d_6)$ values include 2.5—2.9 (m, triphenylmethyl); 3.35 (s, aminothiazole ring proton); 0.6, 1.47, 1.9 (pyridinium protons); 4.3—4.5 (m, C-7 proton); 4.96 (d, J=5Hz, C-6 proton); 8.65 (propyl and t-butyl group protons).

The *title compound* may be deprotected, e.g. as described in Example 13 (b) of US Patent No. 4,258,041 to give (6R,7R) - 7 - [(Z) - 2 - (2 - aminothiazol - 4 - yl) - 2 - (2 - carboxyprop - 2 - oxyimino)acetamido] - 3 - (1 - pyridiniummethyl) - ceph - 3 - em - 4 - carboxylate dihydrochloride.

**Claims**

1. Isolated (Z)-2-(2-t-butoxycarbonylprop-2-oxyimino)-2-(2-triphenylmethylaminothiazol-4-yl)acetyl chloride hydrochloride.

2. The isolated compound according to claim 1 in substantially pure form.

3. The isolated compound according to claim 1 in the form of a free-flowing particulate material.

4. The isolated compound according to claim 1 in crystalline form, which form substantially exhibits the following 'd' values and intensities when subjected to Debye Scherrer X-ray powder diffraction using $CoK\alpha$ and $CuK\alpha$ radiation:—

| 'd' value (Å) | Intensity | 'd' value (Å) | Intensity |
|---|---|---|---|
| 15.55 | s | 4.26 | s |
| 13.71 | vw | 4.10 | w |
| 8.98 | vs | 3.85 | s |
| 6.52 | m | 3.68 | md |
| 6.24 | m | 3.30 | md |
| 5.92 | wd | 3.15 | wd |
| 5.47 | md | 2.87 | wd |
| 4.61 | m | 2.60 | vwd |
| 4.43 | m | 2.54 | vwd |

s=strong; m=medium; w=weak; v=very; d=diffuse.

5. A process for the preparation of the isolated compound according to claim 1 which comprises reacting (Z)-2-(2-t-butoxycarbonylprop-2-oxyimino)-2-(2-triphenylmethylaminotriazol-4-yl)acetic acid or a solvate or salt thereof with a chlorinating agent serving to convert the carboxyl group to a chlorocarbonyl group, hydrogen chloride being liberated in the reaction or otherwise introduced whereby the desired acid chloride hydrochloride is formed and is subsequently isolated.

6. A process according to claim 5 wherein the chlorinating agent is selected from phosphorus pentachloride, phosphorus trichloride and thionyl chloride.

7. A process according to either of claims 5 and 6 wherein the reaction is effected in the presence of an anhydrous organic solvent which is inert under the reaction conditions employed and the desired product is isolated directly from the reaction medium by precipitation, said precipitation being substantially completed by contacting said reaction medium with a further anhydrous organic solvent in which the desired product is less soluble.

8. A process for the preparation of a β-lactam antibiotic compound having a (Z)-2-(2-aminothiazol-4-yl)-2-(2-carboxyprop-2-oxyimino)acetamido side chain which comprises reacting the isolated compound according to any one of claims 1 to 4 with an amino β-lactam compound, followed by removal of protecting groups, whereafter the desired β-lactam antibiotic is recovered.

9. A process according to claim 8 for the preparation of (6R,7R) - 7 - [(Z) - 2 - (2 - aminothiazol - 4 - yl) - 2 - (2 - carboxyprop - 2 - oxyimino)acetamino] - 3 - (1 - pyridiniummethyl)ceph - 3 - em - 4 - carboxylate wherein the starting amino β-lactam compound is 7β - amino - 3 - (1 - pyridiniummethyl)- ceph - 3 - em - 4 - carboxylate or a salt thereof.

**Patentansprüche**

1. Isoliertes (Z) - 2 - (2 - tert. - Butoxycarbonylprop - 2 - oxyimino) - 2 - (2 - triphenylmethylamino- thiazol - 4 - yl) - acetylchlorid-hydrochlorid.

2. Die isolierte Verbindung gemäß Anspruch 1 in im wesentlichen reiner Form.

3. Die isolierte Verbindung gemäß Anspruch 1 in Form eines freifließenden, aus Partikeln bestehenden Materials.

4. Die isolierte Verbindung gemäß Anspruch 1 in kristalliner Form, welche Form im wesentlichen die folgenden 'd'-Werte und Intensitäten aufweist, wenn sie der Debye-Scherrer-Röntgenstrahlen- pulverbeugung unter Verwendung von CoKα- und CuKα-Strahlung unterworfen wird:

## 0 101 148

| 'd'-Werte (Å) | Intensität | 'd'-Werte (Å) | Intensität |
|---|---|---|---|
| 15,55 | s | 4,26 | s |
| 13,71 | vw | 4,10 | w |
| 8,98 | vs | 3,85 | s |
| 6,52 | m | 3,68 | md |
| 6,24 | m | 3,30 | md |
| 5,92 | wd | 3,15 | wd |
| 5,47 | md | 2,87 | wd |
| 4,61 | m | 2,60 | vwd |
| 4,43 | m | 2,54 | vwd |

s=stark; m=mittel" w=schwach; v=sehr; d=diffus.

5. Verfahren zur Herstellung der isolierten Verbindung gemäß Anspruch 1, dadurch gekennzeichnet, daß (Z)-2-(2-tert.-Butoxycarbonylprop-2-oxyimino)-2-(2-triphenylmethylaminothiazol-4-yl)-essigsäure oder ein Solvat oder Salz davon mit einem Chlorierungsmittel, das dazu dient, die Carboxylgruppe in eine Chlorcarbonylgruppe unzuwandeln, umgesetzt wird, wobei Chlorwasserstoff in der Reaktion freigesetzt oder anderweitig eingeführt wird, wodurch das gewünschte Säurechloridhydrochlorid gebildet und anschließend isoliert wird.

6. Verfahren gemäß Anspruch 5, dadurch gekennzeichnet, daß das Chlorierungsmittel ausgewählt ist aus Phosphorpentachlorid, Phosphortrichlorid und Thionylchlorid.

7. Verfahren gemäß einem der Ansprüche 5 und 6, dadurch gekennzeichnet, daß die Reaktion in Anwesenheit eines wasserfreien, organischen Lösungsmittels, das unter den angewandten Reaktionsbedingungen inert ist, durchgeführt wird und das gewünschte Produkt direkt aus dem Reaktionsmedium durch Ausfällung isoliert wird, wobei diese Ausfällung im wesentlichen vervollständigt wird durch Kontaktieren dieses Reaktionsmediums mit einem weiteren wasserfreien, organischen Lösungsmittel, worin das gewünschte Produkt weniger löslich ist.

8. Verfahren zur Herstellung einer β-Lactam-Antibiotikum-Verbindung mit einer (Z)-2-(2-Aminothiazol-4-yl)-2-(2-carboxyprop-2-oxyimino)-acetamido-Seitenkette, dadurch gekennzeichnet, daß die isolierte Verbindung gemäß einem der Ansprüche 1 bis 4 mit einer Amino-β-lactam-Verbindung umgesetzt wird und anschließend die Schutzgruppen entfernt werden, wonach das gewünschte β-Lactam-Antibiotikum gewonnen wird.

9. Verfahren gemäß Anspruch 8 zur Herstellung von (6R,7R) - 7 - [(Z) - 2 - (2 - Aminothiazol - 4 - yl) - 2 - (2 - carboxyprop - 2 - oxyimino) - acetamido] - 3 - (1 - pyridiniummethyl) - ceph - 3 - em - 4 - carboxylat, worin die Ausgangs - Amino - 8 - lactam - Verbindung 7β - Amino - 3 - (1 - pyridinium-methyl) - ceph - 3 - em - 4 - carboxylat oder ein Salz davon ist.

**Revendications**

1. Chlorhydrate de chlorure de (Z)-2-(2-t-butoxycarbonylprop-2-oxyimino)-2-(2-triphénylméthylamino-thiazole-4-yl)-acétyle isolé.

2. Le composé isolé suivant la revendication 1, sous forme essentiellement pure.

3. Le composé isolé suivant la revendication 1 sous la forme d'une matière particulaire fluide.

4. Le composé isolé suivant la revendication 1 sous forme cristalline, laquelle forme présente essentiellement les valeurs "d" et les intensités qui suivant, lorsqu'on la soumet à une difraction des rayons X sur poudre de Debye-Scherrer en utilisant les rayonnements CoKα et CuKα:

| Valeur d (Å) | Intensité | Valeur d (Å) | Intensité |
|---|---|---|---|
| 15,55 | ft | 4,26 | ft |
| 13,71 | tfa | 4,10 | fa |
| 8,98 | tft | 3,85 | ft |
| 6,52 | m | 3,68 | md |
| 6,24 | m | 3,30 | md |
| 5,92 | fad | 3,15 | fad |
| 5,47 | md | 2,87 | fad |
| 4,61 | m | 2,60 | tfad |
| 4,43 | m | 2,54 | tfad |

ft=fort; m=moyen; fa=faible; t=très; d=diffus.

5. Procédé de préparation du composé isolé suivant la revendication 1, caractérisé en ce que l'on fait réagir l'acide (Z)-2-(2-t-butoxycarbonylprop-2-oxyimino)-2-(2-triphénylméthylaminothiazole-4-yl)acétique ou un solvate ou un sel de celui-ci, sur un agent de chloration servant à convertir le radical carboxyle en radical chlorocarbonyle, de l'acide chlorhydrique étant libéré au cours de la réaction ou autrement introduit, si bien que se forme le chlorhydrate du chlorure d'acide souhaité et en ce que l'on isole ensuite ce produit.

6. Procédé suivant la revendication 5, caractérisé en ce que l'on choisit l'agent de chloration parmi le pentachlorure de phosphore, le trichlorure de phosphore et le chlorure de thionyle.

7. Procédé suivant l'une quelconque des revendications 5 et 6, caractérisé en ce que l'on réalise la réaction en présence d'un solvant organique anhydre qui est inerte dans les conditions réactionnelles utilisées et en ce que l'on isole directement le produit souhaité du milieu réactionnel par précipitation, ladite précipitation étant sensiblement achevée par la mise en contact du milieu réactionnel précité avec un solvant organique anhydre supplémentaire dans lequel le produit souhaité est moins soluble.

8. Procédé de préparation d'un composé antibiotique bêta-lactamique, possédant une chaîne latérale du type (Z)-2-(aminothiazole-4-yl)-2-(2-carboxyprop-2-oxyimino)acétamido, caractérisé en ce que l'on fait réagir le composé isolé suivant l'une quelconque des revendications 1 à 4, sur un composé amino bêta-lactamique, cette réaction étant suivie de l'élimination des radicaux protecteurs, puis on récupère l'antibiotique bêta-lactamique souhaité.

9. Procédé suivant la revendication 8 pour la préparation du (6R,7R)-7-[(Z)-2-(2-amino-thiazole-4-yl)-2-(2-carboxyprop-2-oxyimino]acétamido]-3-(1-pyridiniumméthyl)céph-3-ème-carboxylate, caractérisé en ce que le composé amino bêta-lactamique de départ est le 7-bêta-amino-3-(1-pyridinium-méthyl)céph-3-ème-4-carboxylate ou un sel de ce dernier.